# EUROPEAN PATENT SPECIFICATION

(11) **EP 3 288 376 B1**
(45) Date of publication and mention of the grant of the patent: **26.02.2020**
(21) Application number: 16733225.3
(22) Date of filing: 29.04.2016
(51) Int. Cl.: A01K 67/033, B01D 61/14

(54) **HIGH VOLUME BREEDING AND LIFE CYCLE SYNCHRONIZATION SYSTEM**
HOCHVOLUMIGES ZUCHT- UND LEBENSZYKLUSSYNCHRONISATIONSSYSTEM
SYSTÈME DE SYNCHRONISATION DE CYCLE DE VIE ET DE REPRODUCTION EN GRAND VOLUME

(30) Priority: 30.04.2015 NL 2014734; 01.12.2015 NL 2015884
(43) Date of publication of application: 07.03.2018
(73) Proprietor: Millenaar, A.S.B., 5386 AD Geffen (NL)
(72) Inventor: MILLENAAR, Arno, 5345 JX Oss (NL)
(74) Representative: Vogels, Leonard Johan Paul
(86) International application number: PCT/NL2016/050305
(87) International publication number: WO 2016/175658

(56) References cited:
- WO-A1-86/01074
- GANDHI S ET AL: "A simple method for maintaining large, aging populations of Caenorhabditis elegans", MECHANISMS OF AGEING AND DEVELOPMENT, ELSEVIER SEQUOIA, LAUSANNE, CH, vol. 12, no. 2, 1 February 1980 (1980-02-01), pages 137-150, XP023426997, ISSN: 0047-6374, DOI: 10.1016/0047-6374(80)90090-1 [retrieved on 1980-02-01]
- PATEL T R ET AL: "AXENIC AND SYNCHRONOUS CULTURES OF CAENORHABDITIS-ELEGANS", NEMATOLOGICA, BRIL, LEIDEN, NL, vol. 24, no. 1, 1 January 1978 (1978-01-01), pages 51-62, XP009189382, ISSN: 0028-2596
- BYERLY L ET AL: "The life cycle of the nematode Caenorhabditis elegans - II. A simplified method for mutant characterization", DEVELOPMENTAL BIOLOGY, ACADEMIC PRESS, AMSTERDAM, NL, vol. 51, no. 1, 1 July 1976 (1976-07-01), pages 34-48, XP024850268, ISSN: 0012-1606, DOI: 10.1016/0012-1606(76)90120-2 [retrieved on 1976-07-01]
- SONANI RAVI RAGHAV ET AL: "Concurrent purification and antioxidant activity of phycobiliproteins fromLyngbyasp. A09DM: An antioxidant and anti-aging potential of phycoerythrin inCaenorhabditis elegans", PROCESS BIOCHEMISTRY, ELSEVIER, NL, vol. 49, no. 10, 7 July 2014 (2014-07-07), pages 1757-1766, XP029064409, ISSN: 1359-5113, DOI: 10.1016/J.PROCBIO.2014.06.022

## Description

### FIELD OF THE INVENTION

The present invention is in the field of a high volume breeding and life cycle synchronization system for nematodes and a method for breeding such nematodes.

### BACKGROUND OF THE INVENTION

The present invention is in the field of a high volume breeding and life cycle synchronization system for nematodes and a method for breeding such nematodes.

An example of a nematode is Caenorhabditis elegans (C. elegans). C. Elegans is a small, free-living soil nematode (roundworm) that lives in many parts of the world. It feeds mainly on microbes, primarily bacteria. C. Elegans is considered and used as an important "model system" for biological research in many fields including genomics, cell biology, neuroscience and aging (http://www.wormbook.org/); the model system mainly relates to use of a nematode in well-defined and predictable settings and boundary conditions.

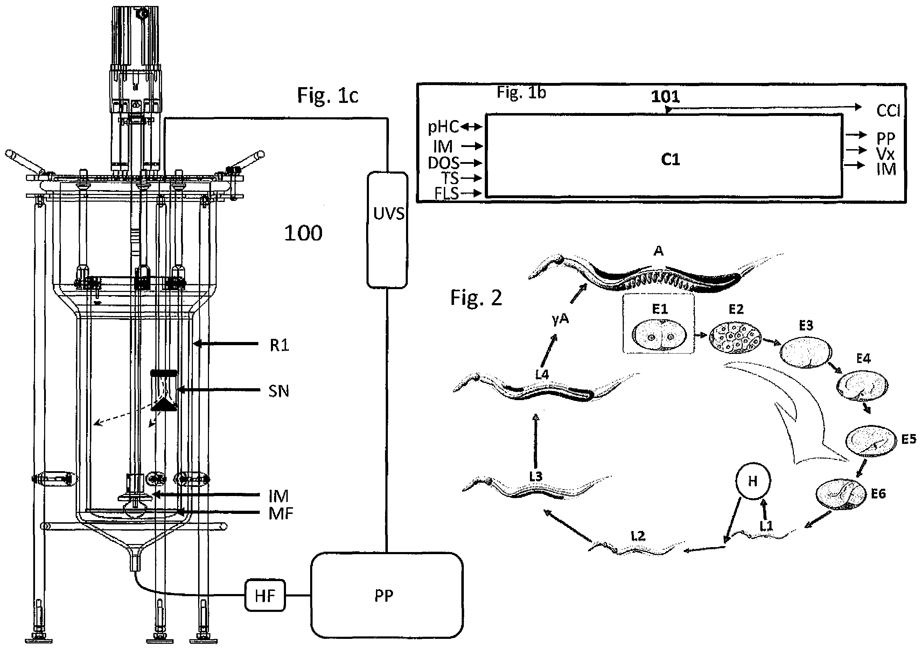

With reference to figure 2 a life cycle of the C. Elegans nematode is presented, which is considered to represent a typical nematode life cycle, i.e. other nematodes have similar life cycles.

The life cycle of hermaphrodite nematode C.elegans takes about 3 days at 20 °C. It is divided into multiple distinguishable stages. In a first stage approximately 250 eggs per adult are laid. The development of the embryo's (embryogenesis) starts at the moment the eggs leave the nematode. Development of the embryo can be divided into different stages, starting at E1 and continued until E6. So called hatching occurs when a fully developed embryo develops from the E6 stage into a L1 larval stage (also referred to as juveniles in some nematode literature). In the case when there is no nutrition available for the L1 larvae, its growth and development will come to a halt (also referred to as arrest) which can last for up to 48 hours (H). If nutrition is presented to the L1 larvae it continues to grow into a L2 larvae, which growth takes about 12 hours. Further development from L2 into L3 takes about 8 hours, whereas development from L3 into L4 takes about 8 hours as well. When L4 larvae continue to grow for another 10 hours they become young Adults (yA). Development from young adult into Adult (A) takes about 8 hours. When the adult stage and form are reached the nematode eggs have developed into the E1 stage. At this point, C. Elegans is capable of laying its first eggs and the cycle may be considered as completed.

In order to obtain accurate and repeatable results in said model system a homogeneous population of the nematode, preferable in its first development stage (L1) or egg stage, is considered a pre-requisite. In order to acquire a homogeneous population of nematodes, e.g. a single stage L1 population, a typical prior art process that is being applied involves bleaching a non-homogeneous population of egg-producing nematodes in order to separate the eggs from the nematode. This process is typically referred to as 'synchronization' or 'staging' in that it is an objective to obtain one single stage (synchronized stage) of the population wherein nematodes have a similar age. Not only is the use of chemicals in the synchronization process a challenging and uncertain process, wherein only a small deviation in temperature, bleach concentration, time, etc. will lead to failure of the egg separation process. In addition the typical yield of the prior art bleaching process is low compared to natural egg producing capabilities of adult nematodes, In addition the L1 nematode hatchlings vitality is negatively affected by the use of the bleaching chemicals, rendering many of the L1 nematodes unsuitable for further research or testing application. In addition to the above drawbacks and rather disappointing yield and quality of the produced L1 nematode, the prior art method is very time consuming and not scalable. Scalability, yield and constant quality are however, for example, a pre-requisite for using the C. elegans nematode in high throughput screening applications.

Incidentally WO 86/01074 A1 recites a growth culture of Entomopathogenic nematodes that can be used as biological insecticides in the fight against certain insect pests. The commercial exploitation of bio-insecticides nematodes has been limited by the inability to grow and produce the nematodes on an industrial and commercial scale. With precise regulation of certain parameters it is therein possible to grow entomopathogenic nematodes in liquid cultures in fermentation tanks, and this is claimed to be on a commercial scale. Minimizing shear forces and by carefully controlling the aeration and agitation, in particular, it is possible to obtain optimal growth of nematodes and their symbiotic bacteria. The population is claimed to be viable, but that is before separation. In addition a full separation into one of various life cycle stages is not shown. Likewise such is the case for a natural environment.

US 8,025,027 (B1) recites an automated insect separation system which processes an aggregate mixture of insects and other materials so that selected insects are separated from the other components of the mix. Specifically, the aggregate mix is directed into a separator apparatus so that the mix flows vertically through a series of vibrating screens. In the preferred embodiment, the screening process separates two different sizes of mealworm larvae from the aggregate mixture. The mix includes the mealworm larvae, unconsumed food materials (usually wheat bran), and insect frass.

WO 02/087321 (A2) recites an apparatus and methods for in vivo mass production of insecticidal nematodes, which the efficiency and volume of nematode production. In the method, nematodes are cultured within a natural insect host. The apparatus comprises at least one harvesting area, a water dispensing system that promotes harvest of nematodes from the host organisms, and a water collection and concentration system for nematode collection and storage. The harvesting area comprises reusable stackable perforated trays, which allow passage of dispensed water while retaining the nematode hosts. The perforations are sized to retain the host organisms and facilitate the passage of harvested nematodes carried within the dispensed water.

DE10215304 (A1)recites an apparatus for selecting invertebrates or vertebrates comprises inducing migration by applying an electric field one or more times. This separates the organisms according to their metabolic state. An Independent claim is included for a method for selecting invertebrates or vertebrates using the apparatus.

Some older articles recite methods for obtaining nematodes. For instance Gandhi et al. In « A simple method for maintaining large, aging populations of C. elegans", in Mechanisms of ageing and Development, Elsevier Sequoia, Lausanne, Vol. 12, No. 2, (February 1980) pp. 137-150, and Patel et al. in "Axenic and synchronous cultures of C. Elegans" in Nematologica, BRIL, Leiden, Vol. 24, No. 1 (January 1978) pp. 51-62, use centrifugation and glass wool or 0.4N NaOH, which may be considered equal to bleaching in terms of aggravation, respectively, for separating nematodes; such will inevitably not result in many viable nematodes and in addition will kill off many others. Gandhi reports on p. 141, 1. 7-10 that only 60-70% of the eggs etch and further hatch relatively unsynchronized during 4-5 hours of incubation. The yield of larvae was even lower. In addition contamination of eggs with worms was still an issue.

The present invention therefore relates to an improved breeding system, and a method of operating said breeding system, which solve one or more of the above problems and drawbacks of the prior art, providing reliable results, without jeopardizing functionality and advantages.

### SUMMARY OF THE INVENTION

The present invention relates to an improved breeding system according to claim 1, a method of operating said breeding system, and describes a population of nematodes obtainable by said method, and use of said population.

The present high volume breeding and life cycle synchronization (HVBS) system can provide relatively high volumes or likewise high amounts of nematodes, such as from 10⁻⁴ litre up to 100 litres or more, in a continuous, semi continuous or batch wise mode of operation. These volumes comprise an aqueous solution forming typically >50% of the volume, and nematodes; a dry weight of the nematodes is typically 1-10% of the nematode volume. For sake of comparison prior art systems typically provide a few millilitres of life cycle synchronized eggs c.q. nematodes, at the most.

An advantage of the present system is that it produces as an output at least twenty times more egg's, i.e. synchronized L1 nematodes, per equivalent amount of bio-mass used as feed supply, compared to prior art methods wherein bleaching is used as a way for synchronization. The biomass of a population may be in the order of 1-10 wt.%, relative to a total mass of the solution, such as 3-5 wt.%. If the present HVBS system continues to operate during a number of consecutive days/cycles, the before mentioned efficiency will further increase to a factor of at least 40-50. The present HVBS system does not destroy the nematode population each and every time when synchronization is required, contrary to prior art methods using bleaching.

It is stressed that the present system does not use harmful or toxic chemicals, such as sodium hypochlorite or bleach, in order to separate the nematode eggs from the population of nematodes. As a consequence it has been found that the present system produces e.g. healthy and synchronized L1 nematodes, suitable for use in research and test applications. There are no negative side effects of the prior art methods and use of bleaching chemicals therein on the vitality of the L1 nematodes observed.

Throughout the description the term "population" refers to i.c. nematodes of at least one life cycle stage being present up to all life cycle stages being present. In a "sub-population" at least one life cycle stage is removed, and sometimes all but one life cycle stages are removed, leaving one life cycle stage left.

The present High Volume Breeder and Synchronizing (HVBS) system as described in this application overcomes drawbacks of the prior art and in addition solves a number of challenges as mentioned below.
1)Synchronization is obtained without the use of chemical(s). In the present system a need for chemicals, other than used for growing the nematodes, is absent. Therewith the nematodes can grow, reproduce, live, and be obtained under favourable conditions.
2)A consistent high quality of output is obtained, i.e. a nematode sub-population, especially eggs and L1 nematodes.

In comparison, prior art methods at the best generate yields of unhatched eggs between 70% and max 90% of eggs being in principle available. Assuming a nematode could produce 250 eggs, prior art method effectively only yields approximately 10 eggs that will hatch, i.e. only 4%. In addition the vitality of the (hatched) nematodes obtained from prior art methods is very much depending on the protocol being used and in many cases a significant percentage is damaged but still 'lives' , rendering the batch of nematodes unusable for the intended purpose. In contrast the present invention provides above 90% (relative to a total number of eggs), typically above 95%, and more typically above 99%, viable nematodes in a repeatable process environment.
3)Breeding, and synchronising is performed in a controlled environment. Such decreases a chance of contamination and it is found easy to monitor and control the growth of C. elegans.
4)Scalability for High Volume use in for example High Throughput Screening applications or research that requires substantial bio-mass.
5)For some systems 24x7 availability 'ready to use' of L1 nematodes.

Thereto the present system uses at least one breeding reactor R1, and incorporated in the reactor at least one micro filter (MF or MFF) over which the content of the breeding reactors is filtered. Filter operation is done in a semi-constant mode or cyclic mode, wherein the fluid content of the breeding reactor is kept in fluid motion through the filter or is flushed over the filter to a second breeding reactor. Two or more breeding reactors may be used, as well as one or more micro filter. In this respect the present system is considered modular. The fluid-motion may be halted/interrupted for filtration and settling purposes. Nematode eggs can then be harvested in an external filter cartridge, which can be easily exchanged. A system may have a number of parallel operating bio-reactors, connected to a control tower. In this respect the present system is considered modular.

The present reactor comprises at least one inlet arranged to receive fluids, and at least one outlet for removing nematodes, optionally a first space between the MF and at least one outlet of the reactor system arranged to receive nematodes, at least one first fluid recirculation connection (FRC), the recirculation connection comprising a nematode harvest micro filter (HF) with a mesh size smaller than the mesh size of the MF, wherein the nematode harvest filter is preferably removably attached to the recirculation connection, wherein the recirculation connection preferably comprises at least one valve (QC1,QC2) which is considered to be equivalent to any other means suited for closing and opening, such as a clamp, preferably a so-called quick coupler with an integrated shut-off, arranged for opening and closing the connection, preferably a nutrient feedstock container (S1) in fluid connection with the breeding reactor, at least one oxygen supply (GS) in fluid connection with the breeding reactor, and a controller (C1) for regulation and controlling operation, such as fluid flow, temperature of the reactor, impellor speed, valve opening and closing, and on/off switching. Valves may also be configured as 3-way valves, selecting or deselecting said (multiple) filters, during the different phases of the process.

In the breeding reactors nutrients and gas, comprising oxygen, may be provided in order to have the nematodes grow, and producing eggs for further generations of nematodes. Thereto a gas supply and a nutrient feedstock are provided. The nutrient feedstock typically comprises bacteria, such as *E. Coli.*

With respect to the present fluid connections it is noted that these may be combined; i.e. the term is mainly intended to indicate that two (or more) elements of the present system are in fluid connection.

In an exemplary embodiment it has been found important to control flow over the MF, in order to have adequate filtering, to prevent clogging of the filter, and in order not to damage nematodes in any life stage. Also the flow of fluid(s) comprising nematodes needs to be controlled, e.g. in terms of throughput. As over the filter fluids are removed from the breeder reactor(s) it is (e.g. in a semi-constant mode) at least sometimes necessary to replenish the amount of fluid in the breeder reactor(s). Thereto a fluid flow through the recirculation connection may be controlled. Thereto a controller is provided.

In an exemplary embodiment a part (1-10% relative) of the fluid is removed from the system; such is repeated regularly. It has been found that by removing part of the fluid a dauer stress hormone is kept at acceptable concentration levels in the fluids, such that it is prevented that nematodes enter a dauer stage. Thereto a removal conduct is provided, comprising a valve V1.

In an exemplary embodiment a high flow rate, will guarantee that eggs can be harvested in the external filter with minimum delay. This again provides a unique advantage to collect (harvest) a batch of eggs within a small time window. An example thereof is that a new filter cartridge can be added and already removed after 15 minutes. By having a relatively small time-window of only 15 minutes, the eggs will all hatch at approximately the same time (within 15-18 minutes) and a perfect natural synchronization will have occurred without the need to withhold nutrients as in some prior-art. By not having to withhold nutrients when the eggs hatch, the nematodes will not be stressed for food and all types of unwanted stress related hormones will be avoided.

It is preferred to use relative to the environment an over-pressure in the present system, in order to prevent air or contaminants from entering the present system.

The system may optionally add a UV sterilization unit, which may be switched on/off, depending on the stage of the process, in the return feed in order to help prevent undesired bacteria, fungi, or even viruses, from entering/remaining in the reactor without the use of chemicals. Using such an UV sterilization device, requires the use of inactivated (not alive) nutrients.

In an exemplary embodiment of system 200 in order to provide a counter pressure over the micro flow filter and to balance an amount of fluid a collector reactor is provided. The collector is also in fluid connection with at least one breeder reactor; the collected fluid and e.g. in case of restarting the present system also nematodes and eggs are returned to the breeder reactor. In order to filter the nematodes properly the collector provides a counter pressure to the micro flow filter. Therewith a pressure over the micro filter can be controlled precisely. The pressure is typically relatively small e.g. to prevent clogging of the filter.

In an exemplary embodiment it has been found important to control pressure differences over the MFF, in order to have adequate filtering, to prevent clogging of the filter, and in order not to damage nematodes in any life stage. Also the flow of fluid(s) comprising nematodes needs to be controlled, e.g. in terms of throughput. As over the filter fluids are removed from the breeder reactor(s) it is (e.g. in a semi-continuous mode) at least sometimes necessary to replenish the amount of fluid in the breeder reactor(s). Thereto a fluid flow between the collector reactor and the breeder reactors may be controlled. Thereto a controller is provided.

It is preferred to use relative to the environment an over-pressure in the present system, in order to prevent air or contaminants from entering the present system.

Thereby the present invention provides a solution to one or more of the above mentioned problems and drawbacks.

Advantages of the present description are detailed throughout the description.

### DETAILED DESCRIPTION OF THE INVENTION

The present invention relates in a first aspect to a breeding system 100 according to claim 1. The present breeding system comprises at least one bio-reactor with an integrated micro-filter. A storage (nutrient feedstock) container will hold nutrients and is connected to the bioreactor, e.g. by means of a controllable peristaltic pump, or likewise a mechanical pump, allowing precise administration of the required amount of nutrient. It is noted that pumping action can be reversed, if required, at least for some of the pumps provided.

In an example at the conical bottom of the bio-reactor vessel and output will lead the filtered fluid output towards an exchangeable filter cartridge. The mesh size of the external filter is chosen such that the nematode eggs are retained, and the nutrients will pass through.

A system may typically be started, also referred to as inoculated, with a small number of nematodes/eggs. In the first start-up phase the system operates with the external filter(s) being removed and the optional UV sterilization unit switched off. After a number of days, depending on the number of nematodes/eggs being used to inoculate the system, the system will reach a level at which the system will contain a sufficient number of nematodes to start the process of harvesting.

In order to prepare the system for harvest mode, it typically is stabilized first, i.e. a homogeneous population of adult nematodes is created. Essentially after a stabilization phase only eggs will be passing through the internal micro filter. In order to reach this point, an external filter will be placed for a period of time in the recirculation connection. The external filter is used for collecting all life-stages of nematodes and eggs, passing through the internal filter, until all nematodes still residing in the bio-reactor are at a life-stage that prevents them from passing through the micro-filter (in view of their size).

When this stage is reached, the output will only contain eggs and no longer contain any other life-stages of the nematodes. The system is now ready for operating in harvest mode. An external filter that may have been used for stabilizing the system, can be disposed or prepared and stored for future inoculation.

The moment the system has stabilized, i.e. only eggs and nutrient being present in the output fluid, an operator, may place an external filter cartridge for harvesting the eggs. Depending on intended use and the quantities c.q. volumes required, the harvest time may be adjusted.

In order to reduce the risk of eggs returning into the bio-reactor, e.g. as a result of operator error, an optional backup filter cartridges may be placed in the return connection, when operating in harvest mode.

After a number of days, typical 2 to 3, the production of eggs will come to a halt as the adult population will have exhausted it capacity to lay eggs. The period the present system will be able to produce eggs may also depends on factors such as temperature and the composition of the different life stages at the time the system was considered stabilized and switched to harvest mode.

When the system is coming to the end of its production cycle, an operator has a choice to re-inoculate the system by harvesting a final batch of eggs or to use a previous harvest of eggs. Typically the existing population of nematodes will be flushed out, before the system will be inoculated again. Inoculation is considered to be a fairly simple process, where a filter cartridge containing previously harvested eggs may be mounted in reverse direction, using a sterile and fresh buffer fluid to flush out the eggs and return them into the bioreactor, while at the same time filling the bioreactor to the required fill level.

In an example of the present breeding system the recirculation connection comprises at least one nematode back-up micro filter with a mesh size smaller than the mesh size of the MF, wherein the at least one back-up nematode filter is preferably removably placed within the recirculation connection.

In an example of the present breeding system the recirculation connection comprises at least one valve per filter arranged for opening and closing the connection, the at least one valve being adjacent to the first nematode filter or to the back-up nematode filter, respectively. Different methods may be used such as a hose clamp in order to remove the filters as required.

In an example of the present breeding system a second space is arranged between the micro filter and a side wall of the reactor in order to allow fluid flow in said second space, and comprising a support for the micro filter.

In an example of the present breeding system the breeding reactor comprises at least one of an air sparger AS to meet biological oxygen demand, a spray nozzle SN for distributing fluids evenly, an impellor IM, an air filter AF, a pressure sensor, a pressure controller, a conical bottom, a fill level sensor, a dissolved oxygen sensor DOS, a pH sensor pHC, a control unit 101/201, and a temperature sensor TS. With the air sparger biological oxygen demand is provided. If an impellor is used preferably a "marine type' blade impellor is used, having a rounded shape, preferable custom designed to be close to the contour of the Micro Filter surface e.g. in system 100, and operating at a low RPM, e.g. in order to prevent damage to nematodes. The impellor may operate in alternating Clock Wise and Counter Clock Wise rotation, depending on operating modus, and may be controlled by the controller. The present reactor preferably has a conical shape, wherein the conical shape is at a bottom thereof. It is preferred to grow nematodes at about 20 °C, thereto a temperature controller including a sensor and not preferred also a heater/cooler is provided. With the air filter an overpressure in the system is feasible, whereas at the same time microbes are prevented from entering the system; an example of the air filter is a bidirectional micro filter with an appropriate mesh size, typically smaller than 1 µm. The air sparger may be placed inside the reactor vessel or integrated in the return fluid connection.

In an example of the present breeding system the at least one micro filter comprises uniform openings (O) with an average opening diameter (d) of 10-100 µm, preferably with an average opening diameter (d) of 40-90 µm, preferably 50-80 µm, more preferably 60-70 µm, such as 63-67 µm. The opening diameter may be adapted in view of a desired population size (cross sectional diameter). Examples are 10-30 µm, such as 20 µm, for only L1 nematodes, 50-80 µm, such as 75 µm, for retaining young adults and adults, and so on up to 100 µm. It has been found that a standard deviation 3σ of <10% relative to the average size is sufficient in this respect, whereas even better results are obtained with a 3σ of <5% relative. The surface area of the MF may vary in view of throughput and/or in view of breeder reactor sizes. Typically the micro filter has a surface area (SA) of 10-5000 cm², such as 50-2000 cm², per plate.

In an example the micro filter mesh plate is attached to a cylinder, with a cylinder diameter smaller than the inner dimension of the bio-reactor vessel. The fluid level within the bio-reactor is typically maintained at a lower level that the top of this cylinder.

At the top of the bio-reactor, a nozzle may spray the returning buffer fluid inside the micro filter cylinder and also partially in-between the micro filter cylinder and the bio-reactor vessel. The fluid that flows on the outside of the micro filter cylinder prevents any eggs or nematodes to get trapped in-between the micro filter cylinder and the bio-reactor vessel and also creates a flow near the bottom of the micro filter cylinder, supporting the eggs to be transported to the output of the bio-reactor. This nozzle and the spray its creates also support oxygen to dissolve into the fluid, making the air sparger optional.

In an example of the present breeding system at least one micro filter comprises a cylindrical section, such as from glass, wherein the cylindrical section extends upwards to a level above a maximum fluid level, and wherein an external diameter of the cylindrical section is 0.5-20 mm smaller than an internal diameter of the reactor, taken at a similar height,

The present internal micro filter may have a polygonal shape, such as hexagonal and octagonal, a circular shape, and an ellipsoidal shape. The micro filter and its filter function may be supported by a rotational movement of the cylinder filter.

In an example the present breeding system comprises a plate section at the bottom of the cylindrical section with openings. Optionally, filter openings may also be present on the vertical section of the cylindrical section, typically towards the lower halve of the cylinder section.

In an example of the present breeding system the plate section is made from a metal, the metal preferably being selected from Ni, stainless steel, Ti, Cr, Si, W, Co, V, Al, and alloys, oxides, and nitrides thereof, and plastic, wherein the plate section preferably has a curvature.

In an exemplary embodiment the external harvest filter cartridge has a filter mesh size, small enough to retain the nematode eggs, however large enough for letting the nutrients pass through. A typical mesh size between 5-10 µm will suffice. It has been found that a standard deviation of <10% relative to the average size is sufficient in this respect, whereas even better results are obtained with a standard deviation of <5% relative. The surface area of the external harvest filter may vary in view of throughput and/or in view of breeder reactor sizes. The harvest filter elements may be made of a polymer, such as PTFE or PVDF, or stainless steel. It may have the form of an exit and inlet tube being connected to a central disc shaped part, or likewise to a tubular shaped part. All components, or some of them, may be disposables. The disposables and likewise the whole system may be cleaned and freed from microorganisms by γ-rays.

In an exemplary embodiment the external harvest filter cartridge may be connected with push button quick coupler connectors in order to facilitate easy change of the filter cartridge and the filters typically will be considered disposable.

In an example the present breeding system further comprises further operational elements, such as one or more pumps, a fill level detector, a dissolved oxygen sensor, a pH sensor and controller, a temperature sensor and temperature controller, and a UV sterilization unit. The pumps may provide pumping action between various elements of the present breeding system. The sensors may be used to determine a status of the present system, e.g. in terms of detection of the fluid level within a reactor. Such information is typically fed back to the controller.

In an example of the present breeding system the impellor IM comprises one or more blades B, preferably 1-6 blades, such as 2, 3, 4 or 5 blades. The present impellor is especially suited for operation at low rpm's, preferably 1-120 rpm, more preferably 2-60 rpm, even more preferably 3-45 rpm, such as 5-30, e.g. 10, 15, and 20 rpm. The impellor comprises an axis having a cross-section cs. The axis may be made from stainless steel. It typically has a diameter of 5-200 mm, such as 50-150 mm, depending on the size of the reactor. The cross-section cs may be a relatively small section of a full cross-section, such as 10-70%, or to a larger part, e.g. 75-100%. The axis has a (virtual) centre point cp and is adapted for connection to a drive in order to provide impelling action. At least one out of mechanical balance blade Bob is provided, that is the impellor may be caused to wobble. The Bob has a (virtual) longitudinal axis LA that does not coincide with the centre point cp, i.e. may be considered "off cp axis", and wherein the at least one blade Bob extents in a direction parallel to the longitudinal axis at least over the cross-section cs of the central axis. It may extent even further such that e.g. 10-30% of the Bob extents on one side, and a remainder, e.g. 65-95%, at the other (or main) side. It is preferred that one or more blades B, including the Bob, are rotated 20-70° with respect to the orientation of the central axis, such as rotated 30-60°. It is preferred that one or more blades (B) are curved with respect to the longitudinal axis, e.g. wherein the curvature forms part of a circle or ellipsoid. It is preferred that one or more blades (B) are shape adapted to follow a contour of the filter, e.g. have a larger width at a central part of the impellor compared to a smaller width at a peripheral part of the impellor.

It has been found that by this special construction also impelling action is provided directly under the cp of the axis, contrary to prior art devices. In addition also an under pressure is created, thereby creating a backflush at the surface of the filter. In view of damage to the nematodes the impellor is preferably operated at low rpm's. By the impelling action clogging of the filter is prevented.

In an example the present breeding system 200 further comprises at least one storage container S2 in fifth fluid connection with the at least one micro flow filter MFF. In a mode of operation the MFF can be deactivated by e.g. closing valves towards the collector. At the same time also flow from a first to a second breeder reactor can be stopped. As a consequence the nematodes in the MFF may consume the nutrients and especially oxygen rather quickly and may starve and/or create a toxic environment. In order to prevent starvation in the MFF, the MFF and its fluid connections can be flushed with a sterile fluid from container S2. As such a steady state situation can be maintained for a few hours, allowing the population of nematodes to further develop into a next stage, or for example allow a homogeneous population of adult nematodes to lay eggs. Thereafter fluid flow from a first to a second breeder reactor can be continued, and filtering over the filter can be effected.

The fluid from the storage container S2 can also be used during the cyclic filtering stages for rinsing the breeder reactors when they are near empty in order to improve the efficiency of the system.

In an example the present breeding system 200 further comprises at least one separator for removing impurities. The separator is intended for purifying the nematode population, especially eggs, in terms of removing impurities. The separator is considered equivalent to the nematode harvest micro filter. Typically impurities present are bacteria, nutrients, and cell residues. The impurities may be separated using a centrifuge, a micro filter, or the like. Typically a 5-20 µm, e.g. 10 µm, pore micro filter is used, allowing impurities to pass through, and maintaining nematodes, especially eggs.

In an example the present breeding system further comprises at least one hatcher reactor and an oxygen supply for the hatcher reactor. The oxygen supply may be the same as for the breeder reactors. In the hatcher reactor e.g. a population of eggs can develop into L1 nematodes in a controlled and precise manner. In addition, by arresting growth, a well-defined population of nematodes and especially L1's may be obtained.

In an example the present system the separator and hatcher reactor are combined into one single device (separator and hatcher), using an integrated mesh filter, allowing rinsing out the impurities, while maintaining the eggs. After rinsing, the eggs remain in the combined device in order to hatch. In addition or as an alternative eggs may after removing impurities be transferred to the final storage containers for hatching and freeing the HVBS for the next batch process; a cycle time may thereby be reduced to an average of about 4 hours.

In an example the present breeding system further comprises a sixth fluid connection between the collector and at least one breeding reactor. The connection provides a return flow opportunity, of which use can be made when stabilizing the present system, for replenishing a fluid amount of the breeder reactor(s), for flushing/cleaning nematodes out of the breeding reactors when in filtering mode, and for returning a mixed population of nematodes and eggs when restarting the system cycle.

In an example the present breeding system further comprises a cleaning unit in fluid connection with at least one element of the present system. In between operations and before operation the present system may need to be cleaned, e.g. into a sterile system. Thereto a cleaning unit is provided. The cleaning unit can clean parts of the present system, e.g. breeding reactors, the MFF, or the full system. The system is preferably equipped with flexible tubes, which may be considered as disposables. The tubes can be removed in between operation and before operation and be replaced. It is preferred to have an integrated cleaning unit, as opening and cleaning of e.g. reactors is cumbersome and time consuming, and opening per se may introduce a risk of (cross-)contamination. As a cleaning medium an alcohol, such as isopropyl alcohol, an alkaline solution, such as NaOH, and combinations thereof can be used. It may be preferred to use a heated solution, e.g. of 60 °C or more. After cleaning and optional replacement of fluid connection tubes the present system is ready for producing nematodes again.

It has been found that complete emptying of the breeder reactor 200 is improved by a conical shape. In order to monitor and control an amount of fluid in the breeder reactor a fill level controller is provided. Typically the reactors are filled from 0% (when empty) to approximately 30%. During the transfer of fluids and nematodes from one reactor R1/R2 to the other R2/R1 the combined volume will add-up to approximately 60% and leave sufficient head-space in order to deal with potential issues such as foaming, etc. A pressure sensor is provided for controlling pressure between the two reactors and over the MFF.

In view of maintaining a uniform flow across all parts of the MFF, thereby avoiding areas where nematodes and alike build-up (clog), certain areas of the filter mesh plate are free from openings, especially corner and side sections thereof. Therefore preferably at least 10% of the surface area does not comprise openings, more preferably at least 20%, and specifically 5-20% relative to a length/width/diameter of the surface at each side thereof is left free from openings. In view thereof the present MFF may have a polygonal shape, such as hexagonal and octagonal, a circular shape, and an ellipsoidal shape.

In an example of the present breeding system the at least one micro flow filter comprises a first plate, the first plate having at least one predefined fluidic path, and at least one inlet and at least one outlet, a filter plate in contact with the first plate, a third plate in contact with the filter plate, the third plate having at least one predefined fluidic path, and an inlet and an outlet, and means for fixing the plates together. Thereto a first plate is provided, typically made of acrylic glass, Poly(methyl methacrylate) PMMA, or the like, having a thickness of 10-30 mm. In the first plate a fluidic path is provided, typically having a depth of 0.2-5 mm. Through inlets the fluid, comprising nematodes and eggs, flows equally over the filter plate. A part of the fluid flows from the first to the second breeder reactor, and a part flows through the filter. Only small nematode sub-populations flow through the filter openings. The small nematodes sub-populations and fluid flow through a fluidic path of a third plate towards an outlet thereof. The third plate has similar characteristics as the first plate. The plates are fixed together using fixing means, therewith providing a liquid tight sealing.

In an example the present breeding system further comprises at least one impurity remover, which may be separate or combined with the hatching bioreactor. In order to arrest growth it is required to remove any nutrients from the L1 hatchlings thereby preventing their further development and as a result creating a synchronized homogeneous population of L1 nematodes. Therefore the impurity separators function is to remove any impurities that could provide nutrients to the hatchlings, i.e. the L1 nematodes after hatching from the eggs.

In an example the present breeding system further comprises further operational elements, such as one or more pumps (MP1-P10), at least one sensor (LS1-LS7) in a fluid connection for detecting the end of the filter cycle, i.e. a fill level of the breeder reactor, and at least one storage reactor (R10,R11,Rx) for storing a sub-population, especially L1 nematodes, preferably a removable storage reactor. The pumps may provide pumping action between various elements of the present breeding system. The sensors may be used to determine a status of the present system, e.g. in terms of detection of empty reactors. Such information is typically fed back to the controller. For practical purpose one or more storage reactors are provided. It is preferred to have a removable reactor R10-Rx; breeding can than take place at a first location, and the use of the sub-population for research and testing and the like at a second location.

For practical purposes, such as costs, volume, etc. various parts of the present system may be combined.

In a second aspect the present invention relates to a method according to claim 13, comprising the steps of (1a)loading the at least one breeding reactor (R1) with an egg producing nematode species, such as C. elegans, (1b)
loading nutrients and oxygen,(2) providing nutrients and oxygen to the at least one breeding reactor (R1),(3)flowing part of the fluid of the at least one breeding reactor through/over the micro filter to an outlet of the reactor, thereby separating early life stage (egg-L4) of the nematode species from (young)adult nematodes thereby creating a homogeneous population of nematodes, and (4)collecting at least one of eggs and other life stages of nematodes, such as in the harvest filter or in the separator. The method relates to a non-bleached production of eggs, with the possibility to use a very short time-window for harvesting the eggs. Using a short time-window will provide a synchronized populations of L1 nematodes, without having to withhold nutrient in order to arrest the development of the hatchlings. It is therefore also no longer required to remove impurities such as nutrients in order to synchronize. As this is a much more natural process c.q. environment for the nematodes, the viability and condition of the hatched L1 nematodes will be optimal as a period of starvation (arrest) as necessary in the prior-art method is no longer required, assuming a relative short time-window will be used when harvesting the eggs.

Based on an example of 1000 ml buffer fluid, stabilizing the system at a level of 1500 nematodes per millilitre a population of approximately 1.5 million nematodes will be available. Assuming each nematode lays between 2 and 6 eggs per hour, the output production rate of such system will be between 3^{∗}10⁶ and 9^{∗}10⁶ eggs per hour. The output quantity ultimately will depend on the amount of fluid and the concentration of gravid nematodes used per millilitre and thereby the overall size of the reactor.

The quality of output of eggs will be close to perfect, i.e. 100% as the process and conditions produce the eggs without the influence of harmful chemicals and even offers the possibility of synchronization without having to apply a period of starvation.

By carefully selecting and adapting breeder system and method conditions the output comprises > 99.99% of unhatched eggs.

In an example of the present method only eggs are collected; the present system provides an option of carefully and precisely selecting a sub-population of nematodes.

In an example the present method comprises the step of (7) harvesting eggs over a period of time in the range of 0.2-540 minutes, preferably 0.5-240 minutes, more preferably 1-120 minutes, even more preferably 2-60 minutes, such as 5-30 minutes.

In an example the present method comprises the step of providing a back-flush over the at least one micro flow filter.

In an example of the present method at some point flowing of the breeder reactor 200 fluid over the mff towards a second reactor starts. The contents of the first breeder reactor are thereby transferred to the second breeder reactor and some fluid is transferred over the filter to e.g. the collector. A differential controlled pressure between the at least two breeding reactors of 1 kPa-200 kPa, preferably 2-100 kPa, more preferably 5-50 kPa, such as 10-20 kPa, is applied. A differential controlled pressure between at least one breeding reactor and collector (R3) of 0.1 kPa-50 kPa, preferably 0.2-20 kPa, more preferably 0.5-10 kPa, such as 1-2 kPa, is applied; such a pressure is considered relatively low. A less preferred alternative one or more peristaltic or mechanical pumps may be used. Therewith an early life stage (egg-L4) of the nematode sub-populations is separated from (young)adult nematodes. A healthy and homogeneous population of adult nematodes is thereby created in the breeder reactors. If the process is continued the population in reactors R1 and R2 becomes more and more homogeneous over time. Using either the sampling output valve SO to determine the output content or by relying on a pre-determined number of filter cycles, the output of the mff will consist mainly (>95%) of eggs. This would be the appropriate moment to switch the output selector valve SW1 towards the filter/impurity separator device IS. The impurity separator could in fact be a combined device with the Egg Hatcher R4. The main purpose of filter IS is to remove the nutrient(s) and other impurities from the eggs. It is considered that when the eggs hatch the L1 stage nematodes should be without nutrients and be prevented from further development. This is referred to as 'arrest'. Than at least one of eggs and L1-L4 nematodes can be collected in the separator.

In an example the present method comprises (10) after removing nutrients and impurities storing the eggs for hatching, e.g. for a period of time < 10 hours. Thereafter the hatchlings, as a homogeneous population, of nematodes (L1), can be stored in at least one storage reactor (R10, R11, R1x). In addition or as an alternative eggs after removing impurities may be transferred to the final storage containers for hatching and freeing the HVBS for the next batch process. As there are no nutrients available for the L1 nematodes to feed on, they will remain 'arrested' and maintain their L1 life cycle development stage. I.e. the nematode population is now synchronized and ready for use.

In an example the present method comprises the steps of (1c) loading a sterile buffer in the buffer container (S2), and after step (3) and before step (4) steps (3ai) wherein the flow is interrupted during a period of 10-600 minutes, during which a sterile buffer is provided to the at least one micro flow filter and the fluid connections between the reactors R1, R2 and R3 respectively, and thereafter repeating step (3), followed by steps (4) and (5). As such the population is further controlled in terms of size distribution and life cycle stage.

In an example the present method comprises the step of providing a back-flush over the at least one micro flow filter from e.g. the collector to at least one breading reactor, thereby cleaning the filter.

In an example of the present method only L1 nematodes or eggs are collected; the present system provides an option of carefully and precisely selecting a sub-population of nematodes.

In an example the present method comprises the steps of (10a) moving to and maintaining the eggs in a hatcher for up to 10 hours under sterile conditions in the absence of nutrients, providing oxygen, and (11a) hatching L1 nematodes. In addition or as an alternative eggs after removing impurities may be transferred to the final storage containers for hatching and freeing the HVBS for the next batch process.

In a third aspect the present invention discloses a non-bleached and synchronized sub-population of nematode eggs. Under the term "bleach" also the term "leach" is considered to fall (treatment with e.g. NaOH), especially in terms of effect on the nematode population. The population does not comprise impurities, contrary to bleaching methods. 99% of the population is not damaged, typically 99.9%, more typically 99.99%; i.e. at the most there is very limited occasional damage. The sub-population typically comprises at least 10² nematodes, such as only one of eggs or L1's, typically E1 eggs, more typically at least 10⁴ nematodes, even more typically at least 10⁶ nematodes, such as 10⁷ nematodes; likewise a volume of nematodes can be provided, such as of 0.001-50 litres; the quantity will depend on the amount of fluid and overall size of the present reactor. By carefully selecting and adapting breeder system and method conditions the sub-population comprises > 90% of only eggs or likewise only L1's ((relative to a total number of living organisms). It has been found that a size distribution of the sub-population can now be well controlled, and the population is healthy; prior art methods at the best generate a sub-population of nematodes of which, after using bleaching to separate the eggs, at the best yields between 70% and max 90% of expected eggs. In addition the present population fully (> 99%) hatches, contrary to prior art methods wherein only 60-70% hatches. Assuming a single nematode could produce 250 eggs, prior art method effectively only yields approximately 10 eggs that will hatch, i.e. only 4% and in many cases will not be viable and thus not suited for further testing/research purposes, e.g. in terms of reliability of the outcome of such testing. The age of the eggs of the population varies only within a time needed to hatch, ±30% of said hatch time, if all synchronized nematodes are hatched. The uncertainty follows from a time before the starting to hatch when e.g. still some eggs may be present in the reactor which eggs have not been flushed over the filter yet. The average residence time of these non-flushed eggs it typically a few minutes, such as 3-5 minutes at the most. The variation in age is therefore typically within ±5% of the hatch time, or better. For example if a synchronized population (eggs) would be harvested within a relative short time window of only 15 minutes, the harvested eggs have an age distribution of 0-15 minutes ±3-5 minutes at the most, the variation merely caused by the time needed to harvest; in this example the age varies with 18-20 minutes at the most. Harvest times may vary from 5-120 minutes, such as 10-60 minutes, or likewise 30 minutes. So all eggs or likewise nematodes have the "same" age, or put different are "born" at "exactly" the same time, within a very small time window. This short time window also avoids unnecessary stress due to the withholding of nutrients as required in prior art method's (i.e. the need for arrest i.e. starvation in order to synchronize). As such using a short time window provides an unprecedented synchronization, not possible with prior art methods. Prior art methods use an incubation time of e.g. 4-5 hours at the best, leading to an age distribution of the same order, e.g. 4-5 hours.

In an example the present sub-population has a size distribution which is characterized by an average size (length) of the nematodes (C. *Elegans)* and a standard deviation 3σ in size of < 30% relative, and typically 3σ in size of < 10% relative, i.e. well defined. For eggs σ in size is even < 2% relative.

In a fourth aspect the present invention discloses a use of a sub-population of nematodes, obtainable by the present method, testing a medicine, for testing a chemical, for testing a substance, for testing toxicity, for testing an agro-chemical, for providing a volume of nematodes, for genomics, for cell biology, for neuroscience, for aging, for phenotyping the population, for providing a population of nematodes, or for high throughput screening.

The one or more of the above examples and embodiments may be combined, falling within the scope of the invention.

### EXAMPLES

The below relates to examples, which are not limiting in nature.

The invention is further detailed by the accompanying figures, which are exemplary and explanatory of nature and are not limiting the scope of the invention. To the person skilled in the art it may be clear that many variants, being obvious or not, may be conceivable falling within the scope of protection, defined by the present claims.

### FIGURES

The invention although described in detailed explanatory context may be best understood in conjunction with the accompanying figures.
Fig. 1a shows an illustration of the present high volume breeding and life cycle synchronization system (100).
Fig. 1b shows an illustration of the Controller Input and Output control signals (101).
Fig. 1c shows a cross-section of the present high volume breeding and life cycle synchronization system (100).
Fig. 2 shows a schematic overview of the life cycle of C. Elegans.

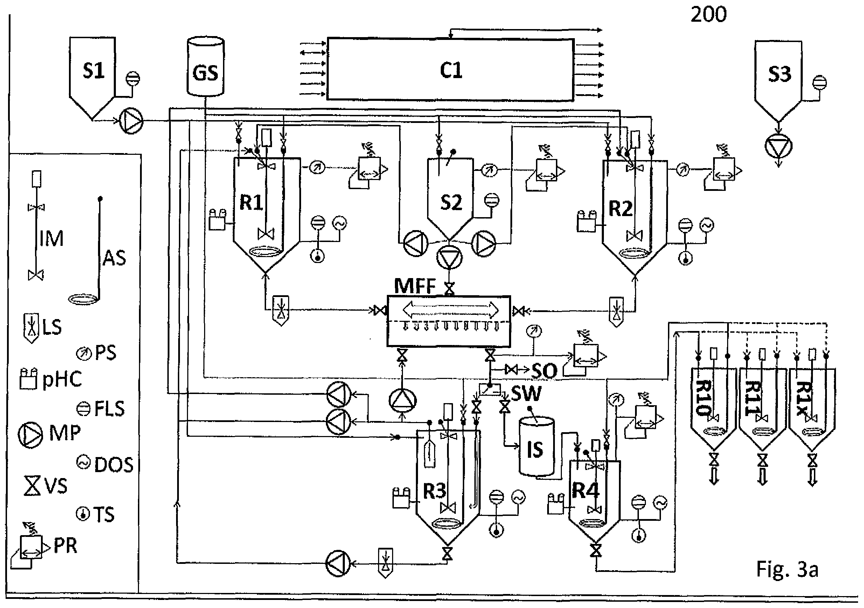

Fig. 3a shows an illustration of the present high volume breeding and life cycle synchronization system (200).
Fig. 3b shows an illustration of the Controller Input and Output control signals (201).
Fig. 4 shows an example of an MFF with two support plates (top + bottom) holding the Micro Mesh Plate in-between. For clarity the assembly is shown in an 'exploded' view without bolts, washers and gaskets.
Fig. 5a-j show schematics of the present impellor.

### DETAILED DESCRIPTION OF THE FIGURES

In the figures:
- 100: HVBS (single reactor)
- 200: HVBS (multiple reactor)
- 101/201: controller (single/multiple reactor)
- AF: air filter
- AS: air sparger
- BF: back-up filter
- Cl: controller
- CCI: Controller Computer Interface
- DOS: dissolved oxygen sensor
- FLS: fluid level sensor
- GS: gas supply
- HF: harvest (cartridge) filter
- IM: impellor
- IS: Impurity separator
- LS: light sensor
- M1: Filter Support Plate with flow channels
- M2: Micro Mesh Plate
- M3: Filter Support Plate with flow channels
- MF: micro filter
- MFF: micro flow filter
- MP: Mechanical pump
- PP: peristaltic (or mechanical) pump
- pHC: pH sensor and controller
- PS: pressure sensor
- PR: pressure regulator
- QCₓ: quick coupler with integrated valve
- RV: regulator valve
- R1,R2: breeding reactor
- R3: collector reactor
- R4: hatcher reactor
- R10: Storage reactor
- R11: Storage reactor
- R1x: storage reactor 1x
- S1: nutrient feedstock container
- S3: Cleaning Fluid Storage container/unit
- SO: Sample output valve
- SN: Spray Nozzle
- SW: Switch valve
- TS: temperature sensor
- Vₓ: valve
- VS: Valve Switch

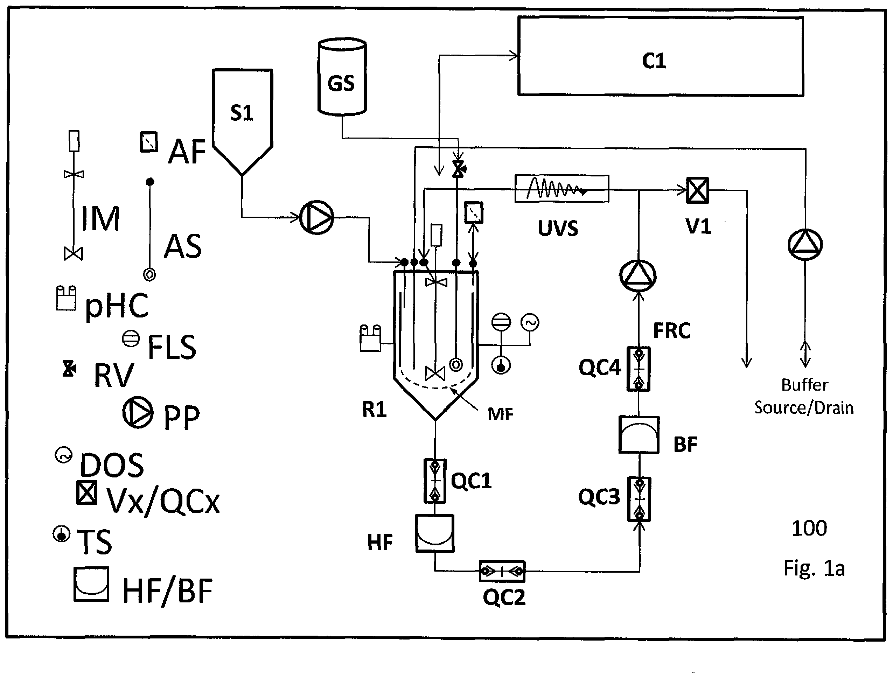

In figure 1c certain elements of the present system are indicated, such as a spray nozzle SN for distributing fluids evenly, an impellor IM, the micro filter MF, a peristaltic pump PP, a UV light source UVS, the reactor R1, and the harvest filter HF.

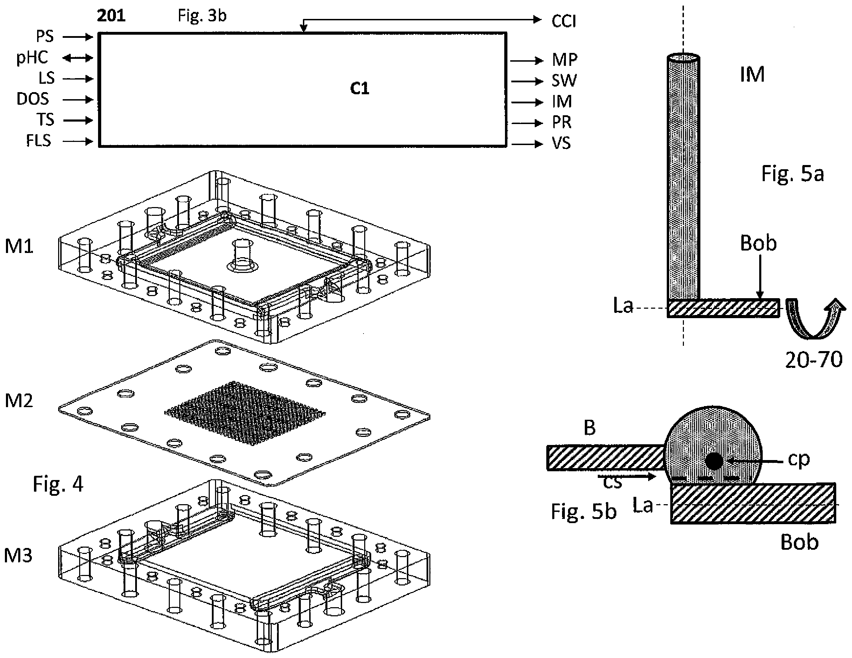

In figure 5a a schematic side view of the present impellor is shown. Therein an axis, having a central virtual axis, is shown. A blade Bob is attached to the axis, typically at a bottom end thereof. The blade is preferably rotated over an angle 20-70° relative to the central virtual axis. The blade has a (virtual) longitudinal axis.

In figure 5b a schematic bottom view of the present impellor is shown. Therein an axis having a central point cp, a blade Bob having a (virtual) longitudinal axis, a second blade B attached in a symmetric manner to the axis, and a cross section cs. The blade Bob may be attached such that the cross section is relatively small, or may be attached close or above the cp, thereby having a large cross section.

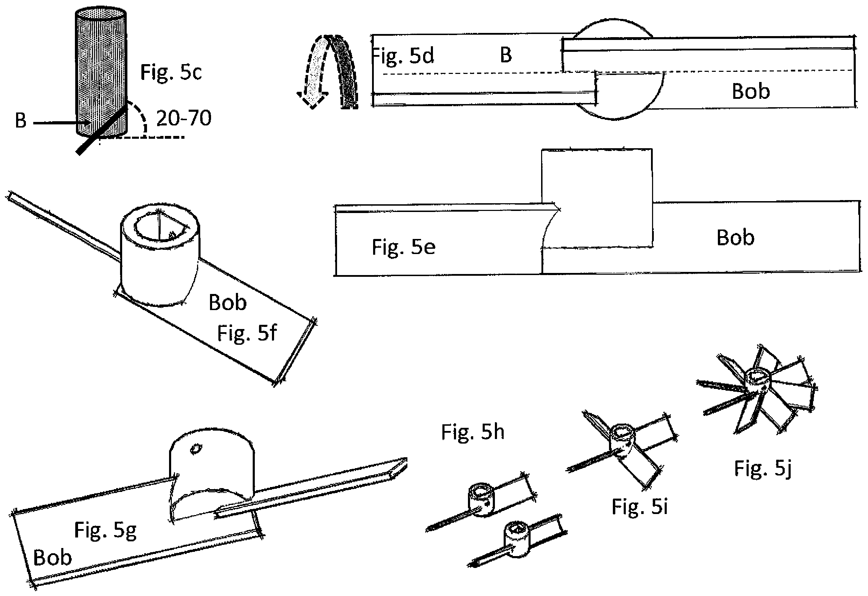

In figure 5c a front view is shown wherein it is shown that the blade B and/or Bob may be rotated an angle 20-70° relative to the central virtual axis.

In figure 5d a bottom view of an impellor with a larger blade Bob and a smaller blade B is shown. The Bob blade is asymmetrical. Both blades extent downwards with respect to the lowest point of the axis. Both blades have a flattened section at a side closest to the bottom (in operation).

In figure 5e a side view of figure 5d is given. In figures 5f and 5g a perspective view of figure 5d is given. In figure 5h a perspective view of the impellor of figure 5d is given; similar views are given in figures 5i and 5j, having four and seven blades respectively.

### HVBS working example (1) description:

After preparation of the HVBS system 100, loading an initial batch of nematodes into the breeding reactor, and allowing time for the nematodes to multiply under optimal and controlled conditions (sufficient nutrients, oxygen, etc.) a next step is to start filtering the content of the breeding reactors.

In the beginning the breeding reactors will contain a non-homogeneous population mix of all levels (stages) of development of nematodes. Filtering is done by mounting an external filter cartridge 'HC', also referred to as the external harvest filter cartridge.

As soon as the external filter cartridge is mounted, the circulation pump will continue and commence filtering. Any nematode life stage small enough to pass through the internal micro filter, will be collected in the external filter cartridge.

After some time, typically within 30 hours, the output of the system will only contain eggs and the system is considered, stable and ready for harvesting eggs.

The filter cartridge used to stabilize the system can be removed and replaced by a new filter cartridge. Depending on the type of synchronization and the output volume required, the filter cartridge will be removed after a set time and the harvested eggs can be used for further processing and use.

### HVBS working example (2) description:

After preparation of the HVBS system 200, loading an initial batch of nematodes into the breeding reactors R1,R2, and allowing time for the nematodes to multiply under optimal and controlled conditions (sufficient nutrients, oxygen, etc.) a next step is to start filtering the content of the breeding reactors. In the beginning the breeding reactors will contain a non-homogeneous population mix of all levels (stages) of development of nematodes. Filtering is done by alternate flushing the content of the two reactors (R1 and R2) over a micro flow filter MFF. By flushing smaller sub-populations, and in a specific example life stages of nematodes smaller than a typical adult size, are filtered out and subsequently collected in collector reactor R3. Multiple filtering cycles, in alternating direction (back and forth), using a controlled pressure difference be-tween the two reactors, are executed. During filtering cycles a controlled counter pressure provided by the collecting third reactor (R3) is maintained. These filtering cycles result in a homogeneous population of adult nematodes in the main reactors R1 and R2, filtering out all smaller sub-populations, such as L1 up to L4 nematodes and eggs, which smaller sub-populations are collected in the R3 collector reactor.

After a number of filter cycles, such as 5-100, de-pending on the volume of the reactors and the characteristics of the micro flow filter used, the main R1 and R2 reactors contain nematodes that have a cross section that is larger than the perforations (or openings) in the micro flow filter. The micro flow filter may contain a mesh. An (aver-age) perforation size of the mesh as used in the micro flow filter is chosen such that only adult and some young adult nematodes will remain in the R1 and R2 reactors.

When the required level of homogeneous population is reached, which is established by little or none L1, L2, L3 and L4 stage nematodes being detected by sampling an output side of the micro flow filter, the micro filter output is switched using a valve switch SW towards an impurity filter system IS. Just before or just after the switch SW a sample tap SO and/or dedicated light sensor may be present, e.g. for determining the content status of the output flow. The IS filter separates the smaller sub-populations, such as the eggs, from any impurities and nutrients, bacteria, and cell residues, before the smaller sub-populations, especially eggs, are being transferred to a hatcher reactor R4. In the hatcher reactor R4 the eggs will be allowed to hatch and evolve into L1 nematodes. A combination of the filter system IS and the hatching reactor R4 is also a feasible option.

After a period of time (up to 10 hours) most of the smaller sub-populations, e.g. eggs, in the hatching storage reactor R4, or optionally in the final storage reactors R10,R11,R1x, will have hatched into the first life stage 'L1' of the nematodes. As there are no nutrients available for the L1 nematodes to feed on, they will remain 'arrested' and maintain their L1 life cycle development stage; i.e. the nematode population is now synchronized and ready for use.

In a final stage the L1 nematodes may be transferred and stored into 'storage bioreactors' R10, R11, Rx. Therein the L1 nematodes can be kept without food (nutrients) for a limited amount of time, while the HVBS system may restart its breeding and/or filtering cycle. In addition or as an alternative eggs may after removing impurities be transferred to the final storage containers for hatching and freeing the HVBS for the next batch process; a cycle time may thereby be reduced to an average of about 4 hours. De-pending on conditions, such as temperature, the 'shelf life' for the L1 nematodes contained in the storage bioreactors is up to 48 hours. In order to maintain a continuous supply of 'fresh' L1 nematodes, the HVBS system is setup to run in a continuous batch-process mode. Therein at regular intervals batched of 'fresh' eggs and/or ready to use L1 nematodes are harvested and transferred to ready to use 'storage bioreactors'.

As mentioned above the HVBS system batch-process is setup and maintained in accordance with a required volume and scheduled use. Multiple final storage bioreactors (R10, R11, R1x) may be required to assure a continuous availability of L1 stage nematodes.

The figures have been detailed throughout the description.

## Claims

1. High volume nematode breeding and life cycle synchronization system (100) comprising
at least one breeding reactor (R1), the at least one breeding reactor having at least one inlet arranged to receive fluids, and at least one outlet for removing nematodes, incorporated in the system, such as in the reactor, at least one micro filter (MF) with filter openings having a mesh size, a first space between the MF and at least one outlet of the reactor system arranged to receive nematodes,
at least one first fluid recirculation connection (FRC) for collecting fluid from the breeding reactor and replenishing fluid to the reactor, the recirculation connection preferably comprising a nematode harvest micro filter (HF) with a mesh size smaller than the mesh size of the MF, wherein the nematode harvest filter is preferably removably attached to the recirculation connection,
wherein the recirculation connection comprises at least one valve (QC1,QC2) arranged for opening and closing a connection, such as the recirculation connection,
preferably a nutrient feedstock container (S1) in fluid connection with the breeding reactor,
at least one oxygen supply (GS) in fluid connection with the breeding reactor, and
a controller (C1) for regulation and controlling operation, such as fluid flow, temperature of the reactor, pH level, impellor speed, valve opening and closing, and on/off switching.

2. Breeding system according to claim 1, wherein the at least one breeding reactor comprises at least one of an air sparger (AS), an air filter (AF), an impellor (IM), a conical bottom, a fill level sensor, a pressure sensor, a pressure controller, a dissolved oxygen sensor (DOS), a pH sensor (pHC), a control unit (101), and a temperature sensor (TS).

3. Breeding system according to any of the preceding claims, wherein the at least one micro filter comprises uniform openings (O) with an average opening diameter (d) of 10-100 µm, and a standard deviation 3σ of <10% relative to the average, wherein the micro filter has a surface area (SA) of 10-5000 cm².

4. Breeding system according to any of the preceding claims, wherein the at least one gas supply provides oxygen.

5. Breeding system according to any of the preceding claims, wherein the recirculation connection comprises at least one second nematode back-up micro filter (BF) with a mesh size smaller than the mesh size of the MF, wherein the at least one second filter is preferably removably attached to the recirculation connection.

6. Breeding system according to any of the preceding claims, wherein the recirculation connection comprises at least one valve (QC) per filter arranged for opening and closing the connection, the at least one valve being adjacent to the first nematode filter or to the second nematode filter, respectively.

7. Breeding system according to any of the preceding claims, wherein a second space is arranged between the micro filter and a side wall of the reactor in order to allow fluid flow in said second space, and comprising a support for the micro filter.

8. Breeding system according to any of the preceding claims, wherein at least one micro filter comprises
a cylindrical section, such as from glass, wherein the cylindrical section extends upwards to a level above a maximum fluid level, and wherein an external diameter of the cylindrical section is 0.5-50 mm smaller than an internal diameter of the reactor, taken at a similar height, and
a plate section at the bottom of the cylindrical section with openings,
wherein the plate section is preferably made from a metal, the metal preferably being selected from Ni, stainless steel, Ti, Cr, Si, W, Co, V, Al, and alloys, oxides, and nitrides thereof, and plastic, wherein the plate section preferably has a curvature, wherein a micro filter surface area preferably comprises openings with an average opening diameter (d) of 15-80 µm.

9. Breeding system (200) according to any of claims 1-4, comprising
at least two breeding reactors (R1,R2) in at least one first fluid connection,
within at least one of the at least one first fluid connection at least one micro flow filter (MFF) with filter openings,
a collector reactor (R3) in third fluid connection with the at least one micro filter, and
wherein the controller (C1) is for regulation and controlling fluid flow and differential pressure, such as (i) at least one fluid flow, (ii) a differential pressure over the at least one micro flow filter, (iii) a differential pressure between at least two of the at least two breeding reactors, and (iv) a fluid flow between the collector reactor and at least one breeding reactor.

10. Breeding system according to claim 9, further comprising at least one of
a storage container (S2) in fifth fluid connection with the at least one micro flow filter (MFF),
a separator (IS),
a hatcher reactor (R4) and an oxygen supply for the hatcher reactor,
a sixth fluid connection between the collector (R3) and at least one breeding reactor (R1,R2),
a cleaning unit (S3) in fluid connection with at least one element of the present system,
a pump (MP1-P10),
a sensor (LS1-LS7) in a fluid connection for detecting a fill level of the breeder reactor, and
a storage reactor (R10,R11,R1x).

11. Breeding system according to claim 9 or 10, wherein at least one micro flow filter comprises
(m1) a first plate, the first plate having at least one predefined fluidic path, at least one inlet, and at least one outlet,
(m2) a filter plate in contact with the first plate,
(m3) a third plate in contact with the filter plate, the third plate having at least one predefined fluidic path, and an inlet and an outlet, and
means for fixing the plates together,
wherein preferably at least 10% of the micro flow filter surface area does not comprise openings.

12. Breeding system according to any of claims 2-11, wherein the impellor (IM) comprises one or more blades (B), preferably 1-6 blades, and an axis having a cross-section (cs) and centre point (cp) adapted for connection to a drive, wherein at least one out of mechanical balance blade (Bob) having a longitudinal axis (LA) that does not coincide with the centre point (cp) and wherein the at least one blade (Bob) extents in a direction parallel to the longitudinal axis at least over the cross-section (cs) of the central axis,
wherein preferably one or more blades (B) are rotated 20-70° with respect to the orientation of the central axis, such as rotated 30-60°,
wherein preferably one or more blades (B) are curved with respect to the longitudinal axis, and
wherein preferably one or more blades (B) are shape adapted to follow a contour of the micro filter.

13. Method of operating a system according to any of the preceding claims, comprising the steps of
(1a) loading the at least one breeding reactor (R1) with an egg producing nematode species, such as C. elegans,
(1b) loading nutrients and oxygen,
(2) providing nutrients and oxygen to the at least one breeding reactor (R1),
(3)flowing part of the fluid of the at least one breeding reactor through/over the micro filter (MF), thereby separating early life stage (egg-L4) of the nematode species from (young)adult nematodes thereby creating a homogeneous population of nematodes, and
(4)collecting at least one of eggs and L1-L4 nematodes such as in the harvest filter or in the separator,
optionally comprising at least one step selected of
(5) harvesting nematodes from the harvest filter, e.g. by removing the filter,
(6) continuing operation until only eggs are produced,
(7) harvesting eggs over a period of time in the range of 0.2-540 minutes,
(8) providing a back-flush over the at least one micro flow filter,
(9) removing nutrients and impurities from the collected at least one of eggs and L1-L4 nematodes,
(10) after removing nutrients storing the eggs for hatching, and
(11) after a period of time < 10 hours storing the population of nematodes (L1) or eggs in at least one storage reactor (R10, R11, R1x),
wherein (3) flowing the fluid of the at least one breeding reactor over the micro flow filter (MFF) is preferably to at least one second breeding reactor, thereby emptying the at least one breeding reactor, and preferably at least once reversing the flow from the at least one second breeding reactor to the at least one breeding reactor, thereby emptying the second breeding reactor, under a differential controlled pressure between the at least two breeding reactors of 1 kPa-200 kPa and a differential controlled pressure between at least one breeding reactor and collector (R3) of 0.1 kPa-50 kPa.

## Patentansprüche

1. Hochvolumiges Nematodenzüchtungs- und Lebenszyklus-Synchronisationssystem (100) umfassend
mindestens einen Züchtungsreaktor (R1), wobei der mindestens eine Züchtungsreaktor mindestens einen Einlass zur Aufnahme von Flüssigkeiten, und mindestens einen Auslass zur Entfernung von Nematoden aufweist, die in das System eingebaut sind, wie beispielsweise in den Reaktor, mindestens ein Mikrofilter (MF) mit Filteröffnungen mit einer Maschengröße, einem ersten Raum zwischen dem MF und mindestens einem Auslass des Reaktorsystems, der zur Aufnahme von Nematoden eingerichtet ist,
mindestens einen ersten Flüssigkeitsrückführungsanschluss (FRC) zum Sammeln von Flüssigkeit aus dem Brutreaktor und zum Nachfüllen von Flüssigkeit in den Reaktor, wobei der Rückführungsanschluss vorzugsweise einen Nematodenernte-Mikrofilter (HF) mit einer Maschengröße aufweist, die kleiner als die Maschengröße des MF ist, wobei der Nematode-Erntefilter vorzugsweise entfernbar an der Rückführverbindung angebracht ist,
wobei die Rückführverbindung mindestens ein Ventil (QC1, QC2) umfasst, das zum Öffnen und Schließen einer Verbindung, wie der Rückführverbindung, angeordnet ist,
vorzugsweise einen Nährstoffbehälter (S1) in Fluidverbindung mit dem Züchtungsreaktor,
mindestens eine Sauerstoffversorgung (GS) in Fluidverbindung mit dem Züchtungsreaktor, und
eine Steuerung (C1) zum Regeln und Steuern des Betriebs, wie z. B. des Fluidstroms, der Temperatur des Reaktors, des pH-Werts, der Geschwindigkeit des Impellors, des Öffnens und Schließens des Ventils, und des Ein- und Ausschaltens.

2. Züchtungssystem nach Anspruch 1, **dadurch gekennzeichnet, dass** der mindestens eine Züchtungsreaktor mindestens einen Luftsprudler (AS), einen Luftfilter (AF), einen Impeller (IM), einen konischen Boden, einen Füllstandsensor aufweist. einen Drucksensor, einen Druckregler, einen Sensor für gelösten Sauerstoff (DOS), einen pH-Sensor (pHC), eine Steuereinheit (101) und einen Temperatursensor (TS).

3. Zuchtsystem nach einem der vorhergehenden Ansprüche, wobei der mindestens eine Mikrofilter uniforme Öffnungen (O) mit einem durchschnittlichen Öffnungsdurchmesser (d) von 10-100 µm, und einer Standardabweichung 3σ<10 aufweist % bezogen auf den Mittelwert, wobei der Mikrofilter eine Oberfläche (SA) von 10-5000 cm² aufweist.

4. Züchtungssystem nach einem der vorhergehenden Ansprüche, wobei die mindestens eine Gasversorgung Sauerstoff liefert.

5. Zuchtsystem nach einem der vorhergehenden Ansprüche, wobei die Rückführverbindung mindestens ein zweites Nematoden-Backup-Mikrofilter (BF) mit einer Maschengröße aufweist, die kleiner als die Maschengröße des MF ist, wobei das mindestens eine zweite Filter vorzugsweise lösbar an der Umwälzverbindung ist angebracht.

6. Züchtungssystem nach einem der vorhergehenden Ansprüche, wobei die Rückführverbindung mindestens ein Ventil (QC) pro Filter umfasst, das zum Öffnen und Schließen der Verbindung angeordnet ist, wobei das mindestens eine Ventil dem ersten Nematodenfilter oder dem ersten Nematodenfilter benachbart ist der zweite Nematodenfilter.

7. Züchtungssystem nach einem der vorhergehenden Ansprüche, wobei ein zweiter Raum zwischen dem Mikrofilter und einer Seitenwand des Reaktors angeordnet ist, um einen Fluidfluss in dem zweiten Raum zu ermöglichen, und einen Träger für den Mikrofilter aufweist.

8. Zuchtsystem nach einem der vorhergehenden Ansprüche, wobei mindestens ein Mikrofilter umfasst
einen zylindrischen Abschnitt, beispielsweise aus Glas, wobei sich der zylindrische Abschnitt bis zu einem Niveau oberhalb eines maximalen Flüssigkeitsstandes nach oben erstreckt und wobei ein Außendurchmesser des zylindrischen Abschnitts 0,5 bis 50 mm kleiner als ein Innendurchmesser des Reaktors ist in ähnlicher Höhe genommen, und
einen Plattenabschnitt am Boden des zylindrischen Abschnitts mit Öffnungen,
wobei der Plattenabschnitt vorzugsweise aus einem Metall hergestellt ist, wobei das Metall vorzugsweise ausgewählt ist aus Ni, rostfreiem Stahl, Ti, Cr, Si, W, Co, V, Al und Legierungen, Oxiden und Nitriden davon und Kunststoff, wobei das Der Plattenabschnitt weist vorzugsweise eine Krümmung auf, wobei eine Mikrofilteroberfläche vorzugsweise Öffnungen mit einem durchschnittlichen Öffnungsdurchmesser (d) von 15 bis 80 um aufweist.

9. Zuchtsystem (200) nach einem der Ansprüche 1 bis 4, umfassend
mindestens zwei Brutreaktoren (R1, R2) in mindestens einem ersten Fluidanschluss,
innerhalb mindestens einer der mindestens einen ersten fluidverbindung mindestens ein mikrodurchflussfilter (mff) mit filteröffnungen,
einen Kollektorreaktor (R3) in dritter Fluidverbindung mit dem mindestens einen Mikrofilter, und
wobei die Steuerung (C1) zum Regeln und Steuern des Fluidstroms und des Differenzdrucks dient, wie (i) mindestens eines Fluidstroms, (ii) eines Differenzdrucks über dem mindestens einen Mikroströmungsfilter, (iii) eines Differenzdrucks Druck zwischen mindestens zwei der mindestens zwei Brutreaktoren und (iv) einem Fluidstrom zwischen dem Kollektorreaktor und mindestens einem Brutreaktor.

10. Zuchtsystem nach Anspruch 9, weiterhin umfassend mindestens eines von
einen Vorratsbehälter (S2) in fünfter Fluidverbindung mit dem mindestens einen Mikrodurchflussfilter (MFF),
ein Trennzeichen (IS),
einen Brütreaktor (R4) und eine Sauerstoffversorgung für den Brütreaktor,
eine sechste Fluidverbindung zwischen dem Sammler (R3) und mindestens einem Brutreaktor (R1, R2),
eine Reinigungseinheit (S3) in Fluidverbindung mit mindestens einem Element des vorliegenden Systems,
eine Pumpe (MP1-P10),
einen Sensor (LS1-LS7) in einer Fluidverbindung zum Erfassen eines Füllstands des Brutreaktors, und ein Speicherreaktor (R10, R11, R1x).

11. Zuchtsystem nach Anspruch 9 oder 10, wobei mindestens ein Mikrodurchflussfilter umfasst
(m1) eine erste Platte, wobei die erste Platte mindestens einen vordefinierten Fluidpfad, mindestens einen Einlass und mindestens einen Auslass aufweist,
(m2) eine Filterplatte in Kontakt mit der ersten Platte,
(m3) eine dritte Platte in Kontakt mit der Filterplatte, wobei die dritte Platte mindestens einen vordefinierten Fluidweg und einen Einlass und einen Auslass aufweist, und
Mittel zum Zusammenhalten der Platten,
wobei vorzugsweise mindestens 10% der Oberfläche des Mikrodurchflussfilters keine Öffnungen umfassen.

12. Zuchtsystem nach einem der Ansprüche 2-11, wobei der Impeller (IM) eine oder mehrere Schaufeln (B), vorzugsweise 1 bis 6 Schaufeln, und eine Achse mit einem Querschnitt (cs) und einem Mittelpunkt (cp) umfasst) angepasst zum Anschluss an einen Antrieb, wobei mindestens eine Unwuchtklinge (Bob) eine Längsachse (LA) aufweist, die nicht mit dem Mittelpunkt (cp) zusammenfällt und wobei die mindestens eine Klinge (Bob) erstreckt sich in einer Richtung parallel zur Längsachse zumindest über den Querschnitt (cs) der Mittelachse,
wobei vorzugsweise eine oder mehrere Klingen (B) um 20 bis 70° in Bezug auf die Ausrichtung der Mittelachse gedreht sind, beispielsweise um 30 bis 60 ° gedreht sind,
wobei vorzugsweise eine oder mehrere Schaufeln (B) bezüglich der Längsachse gekrümmt sind, und
wobei vorzugsweise eine oder mehrere Schaufeln (B) eine Form aufweisen, die einer Kontur des Mikrofilters folgt.

13. Verfahren zum Betreiben eines Systems nach einem der vorhergehenden Ansprüche, umfassend die Schritte von
(1a) das Beladen des mindestens einen Züchtungsreaktors (R1) mit einer eierzeugenden Nematodenart, wie C. elegans,
(1b) Beladen mit Nährstoffen und Sauerstoff,
(2) Zuführen von Nährstoffen und Sauerstoff zu dem mindestens einen Züchtungsreaktor (R1),
(3) das Fließen eines Teils der Flüssigkeit des mindestens einen Züchtungsreaktors durch/über den Mikrofilter (MF), wodurch das frühe Lebensstadium (Ei-L4) der Nematodenart von (jungen) adulten Nematoden getrennt wird, wodurch eine homogene Population erzeugt wird von Nematoden, und
(4) das Sammeln von mindestens einem von Eiern und L1-L4-Nematoden wie im Erntefilter oder im Separator,
optional umfassend mindestens einen Schritt ausgewählt aus
(5) Ernten von Nematoden aus dem Erntefilter, z.B. durch Entfernen des Filters,
(6) Fortsetzung des Betriebs, bis nur noch Eier produziert werden.
(7) Ernten von Eiern über einen Zeitraum im Bereich von 0,2 bis 540 Minuten,
(8) Bereitstellen einer Rückspülung über dem mindestens einen Mikrodurchflussfilter,
(9) Entfernen von Nährstoffen und Verunreinigungen aus den gesammelten Eiern und/oder L1-L4-Nematoden,
(10) nach dem Entfernen von Nährstoffen, die die Bruteier lagern, und
(11) nach einer Zeitspanne von <10 Stunden Lagerung der Population von Nematoden (L1) oder Eiern in mindestens einem Lagerreaktor (R10, R11, R1x),
wobei (3) das Fluid des mindestens einen Züchtungsreaktors über das Mikroströmungsfilter (MFF) vorzugsweise zu mindestens einem zweiten Züchtungsreaktor geleitet wird, wodurch der mindestens eine Züchtungsreaktor geleert wird und vorzugsweise mindestens einmal umgedreht wird strömen von dem mindestens einen zweiten Züchtungsreaktor zu dem mindestens einen Züchtungsreaktor und entleeren dadurch den zweiten Züchtungsreaktor unter einem differenzgesteuerten Druck zwischen den mindestens zwei Züchtungsreaktoren von 1 kPa-200 kPa und einem differenzgesteuerten Druck zwischen mindestens einen Brutreaktor und Sammler (R3) von 0,1 kPa-50 kPa.

## Revendications

1. Système de reproduction et de synchronisation de cycle de vie de nématodes à haut volume (100) comprenant
au moins un réacteur de multiplication (R1), le au moins un réacteur de multiplication ayant au moins une entrée conçue pour recevoir des fluides, et au moins une sortie pour éliminer les nématodes, incorporés dans le système, comme dans le réacteur, au moins un microfiltre (MF) avec des ouvertures de filtre ayant une taille de maille, un premier espace entre le MF et au moins une sortie du système de réacteur agencé pour recevoir des nématodes,
au moins une première connexion de recirculation de fluide (FRC) destinée à collecter le fluide provenant du réacteur de reproduction et de reconstituer le fluide dans le réacteur, la connexion de recirculation comprenant de préférence un microfiltre de récolte de nématodes (HF) avec une taille de maille inférieure à la taille de maille du MF, dans lequel le filtre de récupération de nematode est de préférence fixé de manière amovible à la connexion de recirculation,
dans lequel la connexion de recirculation comprend au moins une vanne (QC1, QC2) agencée pour ouvrir et fermer une connexion, telle que la connexion de recirculation,
de préférence un conteneur de matière première d'éléments nutritifs (S1) en connexion de fluide avec le réacteur de reproduction,
au moins une alimentation en oxygène (GS) en connexion de fluide avec le réacteur de reproduction, et
un contrôleur (C1) pour la régulation et le contrôle du fonctionnement, tel que le débit de fluide, la température du réacteur, le niveau de pH, la vitesse d'imbrouillage, l'ouverture et la fermeture de la vanne et la commutation marche / arrêt.

2. Système de reproduction selon la revendication 1, dans lequel le au moins un réacteur de reproduction comprend au moins un élément parmi un séparateur d'air (AS), un filtre à air (AF), une turbine (IM), un fond conique, un capteur de niveau de remplissage, un capteur de pression, un contrôleur de pression, un capteur d'oxygène dissous (DOS), un capteur de pH (pHC), une unité de commande (101), et un capteur de température (TS).

3. Système de reproduction selon l'une quelconque des revendications précédentes, dans lequel le au moins un microfiltre comprend des ouvertures uniformes (O) avec un diamètre d'ouverture moyen (d) de 10 à 100 µm et un écart type de 3σ<10. % par rapport à la moyenne, le microfiltre ayant une surface spécifique (SA) de 10-5000 cm².

4. Système d'amélioration selon l'une quelconque des revendications précédentes, dans lequel l'au moins une alimentation en gaz fournit de l'oxygène.

5. Système de reproduction selon l'une quelconque des revendications précédentes, dans lequel la connexion de recirculation comprend au moins un deuxième microfiltre auxiliaire pour nématodes (BF) avec une taille de maille inférieure à la taille de maille du filtre MF, dans lequel l'au moins un deuxième filtre est de préférence fixée de manière amovible à la connexion de recirculation.

6. Système de reproduction selon l'une quelconque des revendications précédentes, dans lequel la connexion de recirculation comprend au moins une vanne (QC) par filtre agencée pour ouvrir et fermer la connexion, la au moins une vanne étant adjacente au premier filtre à nématodes ou à le deuxième filtre nématode, respectivement.

7. Système de reproduction selon l'une quelconque des revendications précédentes, dans lequel un deuxième espace est disposé entre le microfiltre et une paroi latérale du réacteur afin de permettre l'écoulement de fluide dans ledit deuxième espace et comprend un support pour le microfiltre.

8. Système de reproduction selon l'une quelconque des revendications précédentes, dans lequel au moins un microfiltre comprend
une section cylindrique, telle que du verre, dans laquelle la section cylindrique s'étend vers le haut jusqu'à un niveau supérieur à un niveau de fluidité maximal, et dans lequel un diamètre externe de la section cylindrique est inférieur de 0,5 à 50 mm à un diamètre interne du réacteur, pris à une hauteur similaire, et
une section de plaque au bas de la section cylindrique avec des ouvertures,
dans lequel la section de la plaque est de préférence constituée d'un métal, le métal étant de préférence choisi parmi le Ni, l'acier inoxydable, le Ti, le Cr, le Si, le W, le Co, le V, le Al et leurs alliages, leurs oxydes et leurs nitrures, et le plastique, dans lequel la section de la plaque a de préférence une courbure, une surface de microfiltre comprenant de préférence des ouvertures avec un diamètre moyen d'ouverture (d) de 15 à 80 µm.

9. Système d'élevage (200) selon l'une quelconque des revendications 1 à 4, comprenant
au moins deux réacteurs de reproduction (R1, R2) dans au moins une première connexion de fluide,
dans au moins une des premières connexions de fluide au moins un filtre à micro-flux (MFF) avec des ouvertures de filtre,
un réacteur collecteur (R3) en troisième liaison fluidique avec le au moins un microfiltre, et
dans lequel le contrôleur (C1) sert à la régulation et au contrôle du débit de fluide et de la pression différentielle, tels que (i) au moins un flux de fluide, (ii) une pression différentielle sur le au moins un filtre à micro-débit, (iii) un différentiel la pression entre au moins deux des au moins deux réacteurs de multiplication, et (iv) un écoulement de fluide entre le réacteur de collecte et au moins un réacteur de multiplication.

10. Système d'élevage selon la revendication 9, comprenant en outre au moins l'un des
un récipient de stockage (S2) en cinquième connexion de fluide avec le au moins un filtre à micro-flux (MFF),
un séparateur (IS),
un réacteur hachoir (R4) et une alimentation en oxygène pour le réacteur hachoir,
une sixième connexion de fluide entre le collecteur (R3) et au moins un réacteur de reproduction (R1, R2),
une unité de nettoyage (S3) en connexion de fluide avec au moins un élément du présent système, une pompe (MP1-P10),
un capteur (LS1-LS7) dans une connexion de fluide pour détecter un niveau de remplissage du réacteur surgénérateur, et
un réacteur de stockage (R10, R11, R1x).

11. Système de reproduction selon la revendication 9 ou 10, dans lequel au moins un filtre à micro-flux comprend
(m1) une première plaque, la première plaque ayant au moins un chemin fluidique prédéfini, au moins une entrée et au moins une sortie,
(m2) une plaque filtrante en contact avec la première plaque,
(m3) une troisième plaque en contact avec la plaque filtrante, la troisième plaque ayant au moins un chemin fluidique prédéfini, ainsi qu'une entrée et une sortie, et
des moyens pour fixer les plaques ensemble,
dans lequel de préférence au moins 10% de la surface spécifique du filtre à micro-flux ne comprend pas d'ouvertures.

12. Système de reproduction selon l'une quelconque des revendications 2 à 11, dans lequel la roue (IM) comprend une ou plusieurs pales (B), de préférence 1 à 6 pales, et un axe ayant une section transversale (cs) et un point central (cp) adapté pour la connexion à un entraînement, dans lequel au moins une pale en équilibre mécanique (Bob) ayant un axe longitudinal (LA) qui ne coïncide pas avec le point central (cp) et dans laquelle l'au moins une pale (Bob) des étendues dans une direction parallèle à l'axe longitudinal au moins sur la section transversale (cs) de l'axe central,
dans lequel, de préférence, une ou plusieurs lames (B) sont tournées de 20 à 70° par rapport à l'orientation de l'axe central, par exemple de 30 à 60°,
dans lequel de préférence une ou plusieurs lames (B) sont courbes par rapport à l'axe longitudinal, et
dans lequel de préférence une ou plusieurs lames (B) sont de forme adaptée pour suivre un contour du microfiltre.

13. Procédé de fonctionnement d'un système selon l'une quelconque des revendications précédentes, comprenant les étapes
(1a) charger au moins un réacteur de reproduction (R1) avec une espèce de nématode producteur d'œufs, telle que C. elegans,
(1b) charge en nutriments et en oxygène,
(2) fournir des nutriments et de l'oxygène à au moins un réacteur de reproduction (R1),
(3) écoulement d'une partie du fluide d'au moins un réacteur de reproduction à travers / sur le microfiltre (MF), séparant ainsi le stade précoce de vie (œuf L4) de l'espèce de nématode des nématodes adultes (jeunes), créant ainsi une population homogènetion de nématodes, et
(4) collecter au moins l'un des œufs et des nématodes L1-L4 tels que dans le filtre de récolte ou dans le séparateur,
comprenant éventuellement au moins une étape choisie parmi
(5) récolter des nématodes à partir du filtre de récolte, par ex. en enlevant le filtre,
(6) poursuivre l'exploitation jusqu'à ce que seuls des œufs soient produits,
(7) récolter des œufs sur une période allant de 0,2 à 540 minutes,
(8) fournir un rinçage à contre-courant sur le au moins un filtre à micro-flux,
(9) éliminer les nutriments et les impuretés de l'un au moins des œufs et des nématodes L1-L4 recueillis,
(10) après avoir éliminé les éléments nutritifs, emmagasiner les œufs et
(11) après une période <10 heures de stockage de la population de nématodes (L1) ou d'oeufs dans au moins un réacteur de stockage (R10, R11, R1x),
dans lequel (3) l'écoulement du fluide d'au moins un réacteur de reproduction sur le filtre à micro-flux (MFF) est de préférence dirigé vers au moins un deuxième réacteur de reproduction, vidant ainsi le au moins un réacteur de reproduction, et de préférence au moins une fois retournant le écoulement du ou des deuxièmes réacteurs d'élevage vers au moins un réacteur d'élevage, vidant ainsi le deuxième réacteur d'élevage, sous une pression différentielle contrôlée entre les au moins deux réacteurs génétiques de 1 kPa à 200 kPa et une pression différentielle contrôlée au moins un réacteur d'élevage et collecteur (R3) de 0,1 kPa à 50 kPa.
